(19) Europäisches Patentamt
European Patent Office
Office européen des brevets

(11) EP 0 646 011 B1

(12) **EUROPEAN PATENT SPECIFICATION**

(45) Date of publication and mention of the grant of the patent:
**09.07.1997 Bulletin 1997/28**

(21) Application number: **93908919.9**

(22) Date of filing: **06.04.1993**

(51) Int. Cl.$^{6}$: **A61K 35/78**

(86) International application number:
**PCT/EP93/00851**

(87) International publication number:
**WO 93/20832 (28.10.1993 Gazette 1993/26)**

# (54) RECTAL PHARMACEUTICAL COMPOSITIONS FOR THE TREATMENT OF PROSTATIC HYPERTROPHY

RECTALE PHARMAZEUTISCHE ZUBEREITUNG FUER DIE BEHANDLUNG VON PROSTATAANSCHWELLUNG

COMPOSITIONS PHARMACEUTIQUES RECTALES DESTINEES AU TRAITEMENT DE L'HYPERTROPHIE DE LA PROSTATE

(84) Designated Contracting States:
**AT BE CH DE DK ES FR GB GR IE IT LI LU MC NL PT SE**

(30) Priority: **10.04.1992 IT MI920873**

(43) Date of publication of application:
**05.04.1995 Bulletin 1995/14**

(73) Proprietor: **PIERRE FABRE MEDICAMENT**
**92100 Boulogne (FR)**

(72) Inventors:
- **FRANCESE, Franco**
  **SmithKline Beecham Farmaceutici**
  **I-20021 Baranzate di Bollate (IT)**
- **OLDANI, Diego**
  **SmithKline Beecham Farmaceutici SpA**
  **I-20021 Baranzate di Bollate (IT)**
- **CROLLA, Tiziana**
  **SmithKline Beecham Farmaceutici**
  **I-20021 Baranzate di Bollate (IT)**
- **VERCESI, Gian, Piero**
  **SmithKline Beecham Farm. SpA**
  **I-20021 Baranzate di Bollate (IT)**

(74) Representative: **Warcoin, Jacques et al**
**Cabinet Régimbeau,**
**26, avenue Kléber**
**75116 Paris (FR)**

(56) References cited:
**EP-A- 0 068 055**
**EP-A- 0 287 000**
**FR-A- 2 480 754**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 646 011 B1

Printed by Rank Xerox (UK) Business Services
2.14.8/3.4

## Description

The present invention relates to rectal pharmaceutical composition for the treatment of prostatic hypertrophy. Particularly, the invention relates to rectal pharmaceutical compositions containing as the active ingredient **Serenoa repens** extract.

**Serenoa repens**, which is also known as **Sabal serrulatum**, is a small palm growing in United States, North Africa and Spain, the lipid-sterol extract of which, obtained according to FR-2480754 or EP-68055 patents, has been used for a long time for the treatment of benign prostatic hypertrophy (prostatic adenoma), prostatitis or other prostatic disorders. The therapeutic scheme used up to now envisages the oral administration of 300 - 1000 mg of extract daily. Therapy must generally be continued for at least two months before an improvement in symptomatology can be attained, and sometimes treatment has to be extended even for 3-4 or more months, in order to obtain a complete remission.

Now it has been found that **Serenoa repens** lipid-sterol extracts, administered by the rectal route in form of soft-gelatin capsules, gives beneficial results in patients suffering from prostatic hypertrophy, prostatitis or related pathologies, in surprisingly shorter times compared with the conventional oral therapy.

According to the invention, a marked improvement in symptomatology can be evidenced already after one week of treatment, whereas the oral treatment requires about two months of treatment to obtain the same results, as proved in clinical trials in which the two different administration routes were compared according to a double blind evaluation scheme.

Therefore, the invention provides pharmaceutical compositions suitable for the rectal administration, containing **Serenoa repens** extracts as the active ingredient admixed with rectally acceptable carriers, in form of soft-gelatin capsules.

The **Serenoa repens** lipid-sterol extract can be formulated, according to the invention, in any form suitable for the enteral or rectal administration, for example suppositories, microclysms, soft-gelatin rectal capsules, rectal ointments, sprays and the like. For this purpose, conventional excipients or carriers and techniques can be used, such as those described in "Remington's Pharmaceutical Science Handbook, Mack Pub. Co., XVII ed, N.Y., USA." As an example, for the formulation of soft-gelatin capsules, an oily dispersion of the active ingredient will be used,obtained using diluents such as plyethylene glycols, vaseline oil, all the vegetable and semi-synthetic officinal oils listed in F.U., Ph. Eur., B.P., USP, Ph. Fr., preferably the medium-chain saturated triglycerids, and dispersants such as polyalcohol fatty acid esters, polyoxyethylene fatty acid esters, polyoxyethylene fatty alcohol esters, polyoxyethylene sorbitane ester derivatives, saccharose fatty acid esters. Saturated polyglycolysated glycerids are preferably used as dispersants.

The formulation can also contain antioxidants, preferably 0.01% butylhydroxyanisole.

After that, the oily dispersion is distributed in a lubrified soft-gelatin shell according to known techniques.

Soft-gelatin capsules containing an oily dispersion of the active ingredient are particularly preferred. The amount of the active ingredient per unit dose will generally range from 50 to 1000 mg, more preferably from 160 to 800 mg.

The compositions of the invention will be administered one or more times daily, depending on the severity of the disorder to treat and on the conditions (weight and age) of the patient.

Generally, the daily dosage will range from about 160 mg to 3 g, but higher or lower dosages can also be envisaged.

Within the dosage ranges indicated above no adverse toxicity effects have been noted.

The following example illustrates the invention.

### Example

### Oily dispersion distributed in soft-gelatin capsules

Formulation of the content of the rectal capsule:

| Component | Amount |
|---|---|
| **Serenoa repens** lipid-sterol extract | 640 mg |
| Medium-chain saturated triglycerids | 100 mg |
| Saturated polyglycolysated glycerids | 100 mg |
| | 840 mg |

### Preparation method

The saturated polyglycolysated glycerids are heated to about 45°C and subsequently diluted, under stirring, with the medium-chain saturated triglycerids.

The dispersion is left to cool to about 30°C, then the **Serenoa repens** lipid-sterol extracts is added, under stirring.

The medicated dispersion is filtered through a 52 microns stainless steel filter and deareated under vacuum.

Finally, the dispersion is filled in soft-gelatin capsules, at a dosage of 840 mg/capsule.

The content is distributed in a lubrified soft-gelatin shell (rectal capsule) prepared according to the conventional technique.

## Claims

**1.** Rectal pharmaceutical compositions containing as the active ingredient a Serenoa repens lipid-sterol extract, in admixture with a rectally acceptable carrier, in form of soft-gelatin capsules.

2. Compositions according to claim 1, containing polyglycolysated saturated glycerids as dispersants.

3. Compositions according to any one of the preceding claims, containing from 50 to 1 000 mg of Serenoa repens lipid-sterol extrat per unit dose.

**Patentansprüche**

1. Pharmazeutische Rectal-Zusammensetzungen, die als aktiven Bestandteil (Wirkstoff) einen Lipid-Sterol-Extrakt von Serenoa repens im Gemisch mit einem rectal akzeptablen Träger in Form von weichen Gelatinekapseln enthält.

2. Zusammensetzungen nach Anspruch 1, die als Dispergiermittel polyglycolysierte gesättigte Glyceride enthalten.

3. Zusammensetzungen nach einem der vorhergehenden Ansprüche, die pro Einheitsdosis 50 bis 1000 mg Lipid-Sterol-Extrakt von Serenoa repens enthalten.

**Revendications**

1. Compositions pharmaceutiques rectales contenant, en tant que principe actif, un extrait lipido-stérolique de Serenoa repens, en mélange avec un véhicule rectalement acceptable, sous forme de capsules molles de gélatine.

2. Compositions selon la revendication 1, contenant des glycérides saturés polyglycolysés en tant que dispersants.

3. Compositions selon l'une quelconque des revendications précédentes, contenant de 50 à 1 000 mg d'extrait lipido-stérolique de Serenoa repens par dose unitaire.